(19) Europäisches Patentamt
European Patent Office
Office européen des brevets

(11) EP 1 656 943 A1

(12) # EUROPEAN PATENT APPLICATION
published in accordance with Art. 158(3) EPC

(43) Date of publication:
**17.05.2006 Bulletin 2006/20**

(21) Application number: **04746382.3**

(22) Date of filing: **18.06.2004**

(51) Int Cl.:
***A61K 36/00*** *(0000.00)* ***A61P 3/08*** *(2000.01)*
***A61P 3/10*** *(2000.01)* ***A61P 5/48*** *(2000.01)*
***A61P 43/00*** *(2000.01)* ***A23L 1/30*** *(1968.09)*
***A23K 1/16*** *(1968.09)* ***C12N 9/99*** *(1980.01)*

(86) International application number:
**PCT/JP2004/008913**

(87) International publication number:
**WO 2004/112817 (29.12.2004 Gazette 2004/53)**

(84) Designated Contracting States:
**AT BE BG CH CY CZ DE DK EE ES FI FR GB GR HU IE IT LI LU MC NL PL PT RO SE SI SK TR**

(30) Priority: **20.06.2003 JP 2003177282**

(71) Applicant: **TAKARA BIO INC.**
**Otsu-shi,**
**Shiga 520-2193 (JP)**

(72) Inventors:
- **OHNOGI, Hiromu**
  **Kusatsu-shi, Shiga 525-0025 (JP)**
- **SUGIYAMA, Katsumi**
  **Otsu-shi, Shiga 520-2134 (JP)**
- **MURAKI, Nobuko**
  **Koka-shi, Shiga 529-1851 (JP)**
- **ENOKI, Tatsuji**
  **Kyotanabe-shi, Kyoto 610-0313 (JP)**
- **SAGAWA, Hiroaki**
  **Kusatsu-shi, Shiga 525-0025 (JP)**
- **KATO, Ikunoshin**
  **Koka-shi, Shiga 529-1851 (JP)**

(74) Representative: **Vossius & Partner**
**Siebertstrasse 4**
**81675 München (DE)**

(54) **EXTRACT FROM PLANT OF JAPANESE PARSLEY FAMILY AND PROCESS FOR PRODUCING THE SAME**

(57) The present invention provides an extract obtainable by extracting a plant belonging to Umbelliferae, or a processed product thereof, with a water-containing alcohol; a process for preparing the extract; a therapeutic agent or prophylactic agent for a disease accompanying an abnormality in an amount of insulin or insulin response and/or diabetic complications, **characterized in that** the therapeutic agent or prophylactic agent comprises as an effective ingredient the extract as defined above; an agent for an insulin-mimetic action,
**characterized in that** the agent comprises as an effective ingredient the extract as defined above; a food, beverage or feed, comprising the extract as defined above; an agent for enhancing glucose uptake into a cell, **characterized in that** the agent comprises as an effective ingredient the extract as defined above; to an agent for inducing differentiation into an adipocyte, **characterized in that** the agent comprises as an effective ingredient the extract as defined above; and an inhibitory agent for aldose reductase, **characterized in that** the agent comprises as an effective ingredient the extract as define above.



Printed by Jouve, 75001 PARIS (FR)

## Description

### TECHNICAL FIELD

The present invention relates to an extract derived from a plant belonging to Umbelliferae richly containing a physiologically active component, a process for preparing the extract, and a medicament, food, beverage or feed containing the extract.

### BACKGROUND ART

*Angelica keiskei* koidz. is a large-scaled perennial herb belonging to Umbelliferae, and a variety of health-promoting effects therefor have been known. For instance, as physiological actions owned by *Angelica keiskei* koidz., anti-bacterial action, anti-tumor action, suppressive action for gastric acid secretion, anti-cancerous effect, enhancing effect for nerve growth factor production, enhancing action for hepatocyte growth factor production and the like have been known (see, for instance, WO 01/76614). However, an insulin-mimetic action such as an anti-diabetic action or anti-obesity action has not been known so far.

Insulin is a hormone necessary for normal metabolism of carbohydrates, proteins and fats in a mammal. Since human suffering from type I diabetes does not sufficiently produce insulin, which is a hormone sustaining life, administration of insulin from the external is required for survival. Human suffering from type II diabetes needs administering with insulin or an agent for enhancing insulin secretion in order to control the glucose level in blood from an inappropriate level, due to causations such as deficiency of the amount of insulin produced and insulin resistance, to an appropriate level. However, among humans suffering from type II diabetes, therapeutic effects may not be found in cases where insulin or the agent for enhancing insulin secretion is administered to diabetic patients of which cause is insulin resistance caused by hyperinsulinemia, abnormality in insulin receptor, aberrance in a downstream signal of the insulin receptor or the like.

In recent years, in order to solve the side effects of insulin and the above-mentioned problems, developments have been made on a substance having physiological functions similar to those of insulin (hereinafter referred to as insulin-mimetic substance in some cases). It has been found that a synthetic benzoquinone derivative is an insulin-mimetic substance (for example, WO 99/51225), and that shikonin derived from *Lithospermum erythrorhizon* is an insulin-mimetic substance (for example, Kamei R. and seven others, *Biochem. Biophys. Res. Commun.,* 2002, Vol. 292, P642-651). These insulin-mimetic substances as mentioned above have been expected to ameliorate symptoms by exhibiting physiological activities similar to those of insulin, not only in type I diabetic patients but also in type II diabetic patients, as well as in type II diabetic patients of which cause is insulin resistance.

### DISCLOSURE OF INVENTION

An object of the present invention is to provide an extract derived from a plant belonging to Umbelliferae richly containing a physiologically active component that is derived from a natural product, safe and conveniently ingestible, a process for preparing the extract, and a medicament, food, beverage or feed containing the extract.

Hereinafter, summarizing the present invention, a first invention of the present invention relates to an extract obtainable by extracting a plant belonging to Umbelliferae, or a processed product thereof, with a water-containing alcohol.

A second invention of the present invention relates to a process for preparing an extract derived from a plant belonging to Umbelliferae or a processed product thereof, comprising the steps of extracting a plant belonging to Umbelliferae, or a processed product thereof, with a water-containing alcohol.

In the first and second inventions of the present invention, the plant belonging to Umbelliferae is preferably exemplified by *Angelica keiskei* koidz. Also, the water-containing alcohol is preferably exemplified by an aqueous ethanol solution having a concentration of 40(v/v)% or more and less than 100(v/v)%.

A third invention of the present invention relates to a therapeutic agent or prophylactic agent for a disease accompanying an abnormality in an amount of insulin or insulin response and/or diabetic complications, characterized in that the therapeutic agent or prophylactic agent comprises as an effective ingredient the extract of the first invention of the present invention.

A fourth invention of the present invention relates to an agent for an insulin-mimetic action, characterized in that the agent comprises as an effective ingredient the extract of the first invention of the present invention.

A fifth invention of the present invention relates to a food, beverage or feed, characterized in that the food, beverage or feed comprises as an effective ingredient the extract of the first invention of the present invention. In the fifth invention of the present invention, the food, beverage or feed is exemplified by a food, beverage or feed for treating or preventing a disease accompanying an abnormality in an amount of insulin or insulin response and/or diabetic complications.

A sixth invention of the present invention relates to an agent for enhancing glucose uptake into a cell, characterized in that the agent comprises as an effective ingredient the extract of the first invention of the present invention.
A seventh invention of the present invention relates to an agent for inducing differentiation into an adipocyte, characterized in that the agent comprises as an effective ingredient the extract of the first invention of the present invention.
An eighth invention of the present invention relates to an inhibitory agent for aldose reductase, characterized in that the agent comprises as an effective ingredient the extract of the first invention of the present invention.

BEST MODE FOR CARRYING OUT THE INVENTION

In the present invention, the plant belonging to Umbelliferae refers to a plant belonging to Umbelliferae of Angiospermae, and the plant belonging to Umbelliferae is exemplified by, for example, *Angelica keiskei* koidz., *Oenanthe javanica, Cryptotaenia japonica Hassk, Angelica pubescens, Daucus, Apium, Petroselium sativum* and the like. In the present invention, *Angelica keiskei* koidz. can be especially suitably used. In addition, the plant belonging to Umbelliferae usable in the present invention is not particularly limited, and fruit, seed, seed coat, flower, leaf, stem, root, rhizome and/or whole plant can be used as it is.
As the raw material for the extract of the present invention, besides the above-mentioned plant belonging to Umbelliferae, a processed product thereof is used. The processed product as used herein is not particularly limited. For example, a powder, a product cut into thin pieces, a dry product, a squeezed juice, or the like of the plant belonging to Umbelliferae can be used. Also, the plant belonging to Umbelliferae may be processed in the form like tea-leaves by a known method, and an extract obtained by subjecting the product to the extraction method of the present invention can be used as the extract of the present invention. In addition, the raw material plant belonging to Umbelliferae may be a mixture of plural plants belonging to Umbelliferae or processed products thereof. In the present invention, each of extracts derived from different plants, extracts derived from different sites of the plant, extracts derived from the same site but extracted with water-containing alcohols having different compositions, or extracts obtained by a combination thereof can be used as the extract of the present invention, alone or in admixture of two or more kinds. Those containing extracts obtained by a different extraction method from the process for preparing the extract of the present invention can be also used as the extract of the present invention as long as the extract of the present invention is contained.
The powder derived from a plant belonging to Umbelliferae can be obtained by, for example, drying the plant, and powdering the product with a powdering machine, or alternatively lyophilizing and powdering the product. The product cut into thin pieces can be obtained by properly cutting a plant belonging to Umbelliferae into thin pieces, and the dry product can be obtained by lyophilization, respectively. The squeezed juice can be obtained by a known method of squeezing a plant belonging to Umbelliferae using, for example, a squeezer of a screw-type, a gear-type, a cutter-type or the like, or a juicer.
In the present invention, the water-containing alcohol is not particularly limited. For example, the water-containing alcohol refers to a mixture of an alcohol such as ethanol, methanol, isopropyl alcohol, ethylene glycol, butylene glycol, propylene glycol, or glycerol, and water (aqueous alcohol solution), which is especially preferably exemplified by a water-containing ethanol. The water-containing alcohol usable in the present invention is, for example, the water-containing alcohol having an alcohol content in terms of its concentration of preferably 40(v/v)% or more and less than 100(v/v)%, more preferably from 45 to 80(v/v)%, especially preferably from 50 to 70(v/v)%, from the viewpoint of being able to even more richly contain the physiologically active substance in the resulting extract, without being particularly limited thereto. Usually, as the water-containing alcohol, it is appropriate to use an aqueous ethanol solution in a concentration of 40(v/v)% or more and less than 100(v/v)%. In addition, these water-containing alcohols can be used alone or as a water-containing alcohol prepared by properly mixing various alcohols, as desired.
In the present invention, the extract refers to a substance obtained through the process of subjecting a raw material plant to an extraction procedure with an extraction solvent. The extraction can be carried out as follows by a known extraction method. For example, a specified site of the raw material plant belonging to Umbelliferae is used as it is or the plant is powdered or cut into thin pieces, and thereafter extracted in a batch process or continuous process using a solvent. The amount of the extraction solvent may be appropriately determined. Usually, the extraction solvent may be used in an amount of the weight of the raw material plant in the form as it is upon use (for example, if the raw material plant is a raw plant, the weight of the raw plant), preferably in an amount of from 0.1- to 100-folds by weight of the raw materials. The extraction temperature may be also appropriately determined according to its purposes, and the extraction temperature is in the range of preferably from 0° to 80°C, more preferably from 0° to 60°C, even more preferably from 0° to 50°C, and especially more preferably from 0° to 40°C. The extraction time may be also determined in consideration of extraction efficiency. It is usually preferable that the raw materials, the extraction solvent and the extraction temperature are set so that the extraction time is within the range of preferably from several seconds to several days, more preferably 5 minutes to 24 hours. The extraction procedure may be carried out, for example, while stirring or allowing the mixture to stand. Also, the extraction procedures may be repeated several times as desired. By the above procedures, an extract derived from the plant belonging to Umbelliferae (hereinafter referred to as the extract of the

present invention in some cases) can be obtained. The extract may be concentrated by subjecting an extract to such a process as filtration, centrifugation, concentration, ultrafiltration or molecular sieving as desired.

For example, when *Angelica keiskei* koidz., one kind of the plant belonging to Umbelliferae, is used as the raw material, the extract of the present invention obtained as described above is a novel extract containing 4-hydroxyderricin and xanthoangelol, which are chalcone compounds having physiological activities, in a high concentration, and further containing two kinds of chalcone compounds found for the first time by the present inventors (hereinafter referred to as TB1 and TB2) in a high concentration. There has not been a report that an extract is obtained from a plant belonging to Umbelliferae using the containment of TB1 or TB2 in a high concentration as an index, and such an extract is obtained for the first time by the present inventors. The chemical structures of TB1 and TB2 are each shown as the following formula (Formula 1):

(Formula 1)

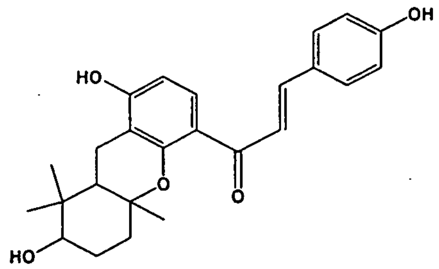

and the following formula (Formula 2):

(Formula 2)

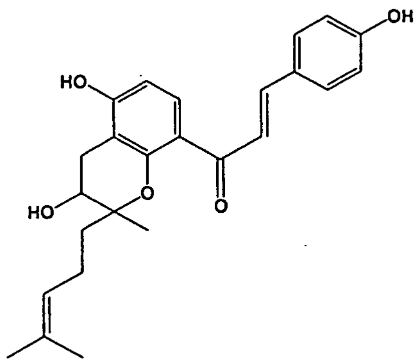

The above-mentioned four kinds of the chalcone compounds, i.e. 4-hydroxyderricin, xanthoangelol, TB1, and TB2, contained in the extract of the present invention are naturally derived insulin-mimetic active components as described in Reference Examples 1 and 2 set forth below. Further, it has been found by the present inventors that TB1 and TB2 have inhibitory actions for aldose reductase, so that their effects on diabetic complications are expected (WO 2004/031165).

Further, a chalcone compound xanthoangelol H, isobavachalcone, or bavachromanol, or a flavanone compound munduleaflavanone A, isobavachin, or prostratol F is contained in the extract of the present invention obtained from *Angelica keiskei* koidz. These compounds have inhibitory actions for aldose reductase and insulin-mimetic actions as shown in Example 11, and Reference Examples 1 and 2 set forth below.

Specifically, the extract obtained by the present invention is very useful as a comprehensive medicament or functional food material for ameliorating or preventing diabetes, which has an effect for preventing diabetes from a normal individual, an effect for ameliorating individuals with a so-called potential for diabetes, which is an earlier stage of diabetes, and an effect for ameliorating diabetes or diabetic complications for individuals who are patients with diabetes and found to show symptoms of complications. Here, the insulin-mimetic action owned by the extract of the present invention is shown in Examples 5 to 7 set forth below using as an index an enhancing action on glucose uptake into a cell or an action of inducing differentiation into an adipocyte. In addition, the aldose reductase inhibitory action of the extract of

the present invention is shown in Examples 8 and 9 set forth below.

In addition, besides the inhibitory action for aldose reductase, since the above-mentioned TB1 and TB2 have a neuronal protection action, a suppressive action for NO production, a suppressive action for interleukin production, or an enhancing action for osteogenesis (see, for example, WO 2004/031165), the extract of the present invention is also useful as a medicament derived from a natural product or functional food material utilizing these physiological activities.

In the present invention, the insulin-mimetic action is not particularly limited as long as at least one of the physiological activities possessed by insulin is exhibited. For example, the insulin-mimetic action is exemplified by at least one metabolic regulatory action selected from enhancement of uptake of a sugar or an amino acid in a cell, and synthesis and degradation inhibition of glycogen or protein. Also, the presence or absence of the insulin-mimetic action can be conveniently determined in accordance with the method described in Example 5 or 6 set forth below. Since the effective ingredient of the present invention has an insulin-mimetic action, there can be exhibited a therapeutic effect or prophylactic effect for all sorts of diseases for which the use of insulin is effective from the therapeutic or prophylactic viewpoint.

In addition, a fraction obtained by fractionating the extract derived from a plant belonging to Umbelliferae of the present invention by a known method, or a fraction obtained by repeating the fractionation procedures a plural times is also encompassed in the extract of the present invention. The above-mentioned fractionation means include extraction, separation by precipitation, column chromatography, thin-layer chromatography, and the like.

In the present invention, the shape of the processed product of the present invention is not particularly limited, and the processed product may take any form of powder, solid or liquid. When the extract is used in the form of powder, a powdery extract of the present invention can be obtained by concentrating the extract of the present invention prepared by extracting the raw material with a water-containing alcohol as a solvent, further adding an excipient or the like thereto, drying and powdering the mixture without being particularly limited thereto. In addition, a granular solid obtained by granulating the extract by a known process can be used as the extract of the present invention. The granulation process is not particularly limited, and is exemplified by tumbling granulation, agitation granulation, fluidizing bed granulation, airflow granulation, extruding granulation, compression molding granulation, disintegration granulation, spray granulation, spray-dry granulation or the like. In addition, the extract in the form of liquid is exemplified by a water-containing ethanol extract itself, a concentrate thereof, a dilution thereof, and an extract made into a liquid form by dissolving the above-mentioned extract in the form of a powder in a liquid, for example water, an alcohol or the like.

In addition, the present invention provides a food, beverage or feed containing the extract of the present invention in a high concentration or at high purity. This means that the food, beverage or feed of the present invention contains a chalcone compound or a flavanone compound as mentioned above in a high concentration and/or at high purity, as compared to that of a conventional food, beverage or feed.

In the present invention, the extract of the present invention may be referred to as the effective ingredient of the present invention, and the therapeutic agent or prophylactic agent for a disease accompanying an abnormality in an amount of insulin or insulin response and/or diabetic complications, wherein the therapeutic agent or prophylactic agent comprises the effective ingredient of the present invention, may be referred to as the therapeutic agent or prophylactic agent of the present invention. Also, the agent for an insulin-mimetic action as well as the therapeutic agent and the prophylactic agent may be collectively referred to as the medicament of the present invention.

No toxicity is especially found in the effective ingredient according to the present invention as mentioned later. Also, there is no risk of the onset of side effects. For these reasons, the disease can be safely and appropriately treated or prevented. Therefore, the therapeutic agent, the prophylactic agent, the food, the beverage or the feed of the present invention, each comprising the effective ingredient, is effective for treating or preventing a disease accompanying an abnormality in an amount of insulin or insulin response and/or diabetic complications.

In the present invention, the disease accompanying an abnormality in an amount of insulin or insulin response includes diseases characterized by a factor selected from change in insulin level in blood, change in activity level of insulin or an insulin receptor, aberrance in downstream signal of an insulin receptor and a combination thereof. The disease is exemplified by, for example, diabetes, obesity, hypertension, arteriosclerosis, cocaine withdrawal symptoms, static cardiac incompetence, amnesia, cardiovascular spasm, cerebral angiospasin, chromaffinoma, ganglioneuroblastoma, Huntington's disease, and hyperlipemia. The diabetes may be exemplified by both type I diabetes and type II diabetes. In addition, the type II diabetes encompasses a disease of which causation is insulin resistance for which a therapeutic effect is not found even when insulin or an agent for enhancing insulin secretion is administered.

The disease accompanying an abnormality in an amount of insulin or insulin response is more likely to lead to deficiency in the amount of insulin production, or deficiency in the action of insulin caused by insulin resistance in the onset stage of the disease. Since the effective ingredient of the present invention can suppress the onset of the disease accompanying an abnormality in an amount of insulin or insulin response by showing an insulin-mimetic action, the prophylactic effects of the disease can also be expected, and the prophylactic effects of the diabetic complications can also be expected.

In the state of insulin resistance, a signal from an insulin receptor by insulin is inhibited, so that diversified

functions possessed by insulin are not exhibited, whereby generating various dysbolisms. The effective ingredient used in the present invention can exhibit an insulin-mimetic effect also on a symptom of insulin resistance. Specifically, by using the prophylactic agent or therapeutic agent of the present invention, a therapeutic or prophylactic effect can be exhibited also for a disease caused by insulin resistance, for example, type II diabetes for which a therapeutic effect is not seen even when insulin or an agent for enhancing insulin secretion is administered. In addition, the effective ingredient of the present invention can also exhibit the effect of lowering the amount of insulin in blood. In other words, the medicament of the present invention can be also used as a therapeutic agent or prophylactic agent for a disease requiring lowering of the amount of insulin for treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature,* vol. 424, P277-284 (2003)), the medicament of the present invention can also be used as an agent for anti-aging.

There have been known that insulin enhances induction of differentiation of preadipocytes into adipocytes, and that glucose uptake takes place in matured adipocytes thereby accumulating triglyceride in the adipocytes *(J. Biol. Chem.,* Vol. 253, No. 20, P7570-7578 (1978)). Specifically, utilizing this finding, an insulin-mimetic action of a test substance can be determined by administering a test substance in place of insulin, and determining differentiation into an adipocyte and the amount of triglyceride in the adipocytes.

In addition, there been known that insulin has an enhancing action on glucose uptake into a cell, and that glucose uptake into the cell is enhanced by the action of insulin in a matured adipocyte *(J. Biol. Chem.,* Vol. 253, No. 20, P7579-7583 (1978)). Specifically, utilizing this finding, an insulin-mimetic action of a test substance can be determined by administering a test substance in place of insulin, and determining the amount of glucose uptake into a matured adipocyte.

In the present invention, the diabetic complications are exemplified by, for example, diabetic retinopathy, diabetic peripheral nerve diseases, diabetic neurosis such as cataract, deafness, paresthesia, and muscular atrophy; diabetic nephropathy such as renal failure; diabetic vascular diseases such as arteriosclerosis and cerebral infarction; infections caused by lowering of phagocytic action of leukocytes; diabetic coma; and the like.

The therapeutic agent or prophylactic agent of the present invention includes ones formed into a preparation by combining the above-mentioned effective ingredient according to the present invention with a known pharmaceutical carrier. Also, as the therapeutic agent or prophylactic agent of the present invention, the above-mentioned effective ingredient can be combined with other components which can be used for the same applications as those of the effective ingredients, for example, an insulin preparation, an agent for enhancing insulin secretion, an agent for improving insulin resistance, an agent for ameliorating postprandial hyperglycemia, an agent for an insulin-mimetic action, an inhibitory agent for aldose reductase, or the like which is known in the art.

The therapeutic agent or prophylactic agent of the present invention is usually manufactured by combining the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like can be optionally added thereto, to form a solid agent such as a tablet, a granule, a powder, an epipastic, and a capsule, or a liquid agent such as a common liquid agent, a suspension agent or an emulsion agent. In addition, there can be also formed into a dry product which can be liquefied by adding an appropriate carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for example, starch, lactose, saccharose, mannitol, carboxymethyl cellulose, cornstarch, an inorganic salt or the like. In addition, upon preparation of the orally administered preparation, a binder, a disintegrant, a surfactant, a lubricant, a fluidity accelerator, a corrective, a colorant, a flavor, and the like can be further combined therewith. In the case of forming into a tablet or a pill, for example, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for example, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a parenterally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, a tonicity agent, a soothing agent, or the like if needed. It is also possible to produce a solid composition and dissolve the composition in sterile water or a sterile solvent for injection before use.

The external preparation includes solid, semi-solid or liquid preparations for percutaneous administration or transmucosal (intraoral or intranasal) administration. The external preparation also includes suppositories and the like.

For example, the external preparation may be prepared as liquid preparations including emulsions, suspensions such as lotions, external tinctures, and liquid agents for transmucosal administration; ointments such as oily ointments and hydrophilic ointments; patches for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is preferably in an amount so that the effective ingredient can be administered within the dose range described below in consideration of administration form, administration method and the like of the preparation. The content of the effective ingredient in the therapeutic agent or prophylactic agent of the present invention is usually from 0.1 to 100% by weight or so.

The therapeutic agent or prophylactic agent of the present invention is administered via an administration route appropriate for each of the preparation form. The administration method is also not limited to specific one. The agent can be administered internally, externally (or topically) and by injection. The injection can be administered, for example, intravenously, intramuscularly, subcutaneously, intracutaneously, or the like. As to an external preparation, for example, a suppository may be administered according to its proper administration method.

The dose of the therapeutic agent or prophylactic agent of the present invention is changeable and properly set depending upon its preparation form, administration method, purpose of use, and age, weight, symptom or the like of a patient to which the therapeutic agent or prophylactic agent is administered. Generally, the dose of the agent, in terms of the dose of the above-mentioned effective ingredient contained in the preparation, on a dry basis, is preferably from 0.001 mg to 10 g/kg weight, and more preferably from 0.1 mg to 1 g/kg weight, per day for adult. As a matter of course, the dose varies depending upon various conditions, so that an amount smaller than the dose mentioned above may be sufficient, or an amount exceeding the dose range may be required. Administration may be carried out once or in several divided portions in a day within the desired dose range. The administration period may be arbitrarily determined. Also, the therapeutic agent or prophylactic agent of the present invention can be orally administered as it is, or the agent can be added to any foodstuffs to be taken on a daily basis.

In addition, the present invention can provide an agent for an insulin-mimetic action comprising the above-mentioned effective ingredient. The agent for an insulin-mimetic action may be the above-mentioned effective ingredient itself, or a composition comprising the above-mentioned effective ingredient. The agent for an insulin-mimetic action may be prepared by, for example, combining the above-mentioned effective ingredient with other component, for example, an insulin preparation, an agent for enhancing insulin secretion, an agent for improving insulin resistance, an agent for ameliorating postprandial hyperglycemia, an agent for an insulin-mimetic action or the like which is known in the art, which can be used for the same application as that of the effective ingredient, and forming into a form of reagent usually used according to the above-mentioned process for preparing the therapeutic agent or prophylactic agent. The content of the above-mentioned effective ingredient in the agent for an insulin-mimetic action is not particularly limited, as long as the content is in an amount so that the desired effects of the present invention can be exhibited in consideration of administration method, purpose of use or the like of the agent for an insulin-mimetic action. A typical content of the effective ingredient in the agent for an insulin-mimetic action of the present invention is from 0.1 to 100% by weight or so. Also, the amount of the agent for an insulin-mimetic action used is not particularly limited, as long as the desired effects of the present invention can be exhibited. Especially in the case where the agent for an insulin-mimetic action is administered to a living body, the agent for an insulin-mimetic action may be preferably used in an amount so that the effective ingredient can be administered within the dose range of the effective ingredient in the above-mentioned therapeutic agent or prophylactic agent. The agent for an insulin-mimetic action is useful in a disease accompanying an abnormality in an amount of insulin or insulin response. In addition, the agent for an insulin-mimetic action is useful in screening of drugs for diseases accompanying an abnormality in an amount of insulin or insulin response. Furthermore, the agent for an insulin-mimetic action is useful in studies on mechanisms of an action of insulin on cells, or functional studies relating to physical changes in the cells. In addition, the agent for an insulin-mimetic action can be used by adding the agent in place of or together with serum or insulin preparation to a medium for culturing a cell, a tissue, or an organ. The medium is very useful as a medium for culturing a cell, a tissue or an organ that has reduced level of, or contains no serum or insulin preparation.

In addition, administration of the agent for an insulin-mimetic action of the present invention to human can be expected to lower the amount of insulin in blood. In other words, the agent for an insulin-mimetic action of the present invention can also be used as a therapeutic or prophylactic agent for a disease requiring the lowering of the amount of insulin for the treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature,* vol. 424, P277-284 (2003)), the agent for an insulin-mimetic action of the present invention can also be used as an agent for anti-aging.

No toxicity is especially found in the effective ingredient according to the present invention as described later. Also, there is no risk of the onset of side effects. For these reasons, the insulin-mimetic action and/or the inhibitory action

for aldose reductase can be safely and appropriately exhibited in a living body. Therefore, the medicament, food, beverage or feed of the present invention comprising the effective ingredient is effective for a disease accompanying an abnormality in an amount of insulin or insulin response, and/or diabetic complications.

In addition, the present invention provides a food, beverage or feed comprising the extract of the present invention. Since the food, beverage or feed of the present invention has an insulin-mimetic action or an inhibitory action for aldose reductase, the food, beverage or feed is very useful in amelioration of symptoms or prevention of a disease accompanying an abnormality in an amount of insulin or insulin response, or diabetic complications. Furthermore, the food or beverage of the present invention is a food or beverage for lowering blood sugar level, having the action of lowering a blood sugar level, so that the food or beverage is useful as a functional food or beverage effective for an individual who cares about his/her blood sugar level or an individual who cares about his/her body fat.

The food, beverage or feed of the present invention can be combined with another substance which is known to have an anti-diabetic action, for example, a substance having an insulin-mimetic action, a substance having enhancing action on insulin secretion, a substance having an action of improving insulin resistance, a substance having an action of ameliorating postprandial hyperglycemia, or an inhibitory substance for aldose reductase, which is commonly known. For example, the food, beverage or feed can be combined with hardly digestible dextrin or the like.

The term "comprising" in the food, beverage or feed of the present invention encompasses the meanings of containing(ed), adding(ed) and/or diluting(ed). As used herein, the term "containing(ed)" refers to an embodiment of containing the extract of the present invention in the food, beverage or feed; the term "adding(ed)" refers to an embodiment of adding the extract of the present invention to a raw material for the food, beverage or feed; and the term "diluting(ed)" refers to an embodiment of adding a raw material for the food, beverage or feed to the extract of the present invention.

The process for preparing the food, beverage or feed of the present invention is not particularly limited. For example, combination, cooking, processing, and the like can be carried out in accordance with those generally employed for foods, beverages or feeds, and the food, beverage or feed of the present invention can be prepared by the general methods for preparing a food, beverage or feed, as long as the resulting food, beverage or feed contains the extract of the present invention.

The food or beverage of the present invention is not particularly limited. The food or beverage includes, for example, processed agricultural and forest products, processed livestock products, processed marine products and the like, including processed grain products such as processed wheat products, processed starch products, processed premix products, noodles, macaronis, bread, bean jam, buckwheat noodles, wheat-gluten bread, rice noodles, *fen-tiao,* and packed rice cake; processed fat and oil products such as plastic fat and oil, tempura oil, salad oil, mayonnaise, and dressing; processed soybean products such as tofu products, soybean paste *(miso),* and fermented soybeans; processed meat products such as ham, bacon, pressed ham, and sausage; marine products such as frozen ground fish, steamed fish paste, tubular roll of steamed fish paste, cake of ground fish, deep-fried patty of fish paste, fish ball, fascia and tendon, fish meat ham, sausage, dried bonito, products of processed fish egg, canned marine products, and preserved food boiled down in soy sauce *(tsukudani);* milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor *(shochu),* vodka, brandy, gin, rum, beer, refreshing alcoholic beverages, fruit liquor, and liqueur; luxury drinks such as green juice *(aojiru),* green tea, tea, oolong tea, coffee, refreshing beverages and lactic acid beverages; seasonings such as soy sauce, sauce, vinegar, and sweet rice wine; canned, bottled or pouched foods such as rice topped with cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods such as soups; spices; and the like, in which each of the foods and beverages comprises the extract of the present invention.

In the food of the present invention, the extract of the present invention is contained, added and/or diluted, alone or in plurality, and its shape is not particularly limited. The shape includes those which can be taken orally such as tablets, granules and capsules.

As the process for preparing a tablet-shaped food, the tablet-shaped food can be prepared by a known process without particular limitation. The tablet-shaped food can be prepared by, for example, extracting a dry powder of *Angelica keiskei* koidz. with a 60(v/v)% aqueous ethanol solution, adding an excipient, for example, dextrin, hardly digestible dextrin, cornstarch, tapioca, cyclodextrin or the like to a concentrated extract, lyophilizing and powdering the mixture, to give a dry powder of an extract from *Angelica keiskei* koidz., thereafter further properly mixing therewith lactose, crystalline cellulose, a sucrose fatty acid ester, a reducing maltose, calcium phosphate, egg shell calcium, particulate

silicon dioxide, CMC-Ca, trehalose, various vitamins such as vitamin C, citric acid, a sweetener such as glucose, a sugar, a sugar alcohol, or stevia, a flavor, a powder juice, a marine alga-derived powder (for example, fucoidan-containing powder, agarooligosaccharide powder, or the like), a mushroom powder, a dried vegetable powder (for example, a powder of a plant belonging to Umbelliferae such as *Angelica keiskei* koidz.), or the like, optionally subjecting the mixture to a granulation process, and tableting the mixture with a rotary tableting machine.

Also, as the process for preparing a granular food, the granular food can be prepared by a known process without particular limitation. The granular food can be prepared by, for example, adding ethanol to various kinds of mixed powders obtained before subjecting to the rotary tableting machine in the process for preparing the above-mentioned tablet-shaped food, kneading the mixture together, granulating the mixture with an extrusion granulator, drying the resulting granules, and sizing the granules with vibration sieves.

In addition, as the process for preparing a capsule-shaped food, the capsule-shaped food can be prepared by a known process without particular limitation. The capsule-shaped food can be prepared by, for example, packing various kinds of mixed powders obtained before subjecting to the rotary tableting machine in the process for preparing the above-mentioned tablet-shaped food into No. 1 Capsule. Alternatively, a glycerol fatty acid ester, beeswax or the like is added to the powder before packing to emulsify the mixture, and a soft capsule can be produced using gelatin and glycerol as the coating materials.

Here, the extract of the present invention has lowered acerbity and astringency peculiarly owned by the plant belonging to Umbelliferae, so that the extract is also excellent as a food raw material. In other words, the food or beverage of the present invention is a food or beverage excellent in palatability.

In addition, the beverage of the present invention can be prepared by a known process without particular limitation. The beverage of the present invention can be prepared by, for example, extracting a dry powder *of Angelica keiskei* koidz. with a 60(v/v)% aqueous ethanol solution, adding an excipient, for example, dextrin, hardly digestible dextrin, cornstarch, tapioca, cyclodextrin or the like to a concentrated extract, lyophilizing and powdering the mixture, to give a dry powder of an extract from *Angelica keiskei* koidz., thereafter further properly adding thereto a flavor, a sweetener, a fruit juice, a powder fruit juice, a vegetable extract, a vegetable paste, a marine alga-derived powder or a marine alga-derived extract (for example, fucoidan-containing product, agarooligosaccharide-containing product, or the like), a mushroom powder, a dry powder of a plant belonging to Umbelliferae, or the like, and dissolving the mixture in water or an alcohol. Alternatively, the beverage of the present invention can be prepared by dissolving a dry powder of an extract from *Angelica keiskei* koidz. in water, and thereafter preparing the beverage in accordance with the process for preparing a known refreshing beverage. In addition, a concentrate of the extract from *Angelica keiskei* koidz. can be used in place of the dry powder of the extract from *Angelica keiskei* koidz. Further, an alcoholic beverage can be prepared as the beverage of the present invention using an extract from *Angelica keiskei* koidz. with a water-containing alcohol.

The content of the above-mentioned effective ingredient in the food or beverage of the present invention is not particularly limited, and the content can be appropriately selected from the viewpoints of sensory aspect and exhibition of activity. The content of the above-mentioned effective ingredient is, for example, preferably 0.00001% by weight or more, more preferably from 0.0001 to 90% by weight, and even more preferably from 0.0006 to 80% by weight, on a dry basis, of the food. The content is, for example, preferably 0.00001% by weight or more, more preferably from 0.0001 to 90% by weight, and even more preferably from 0.0006 to 80% by weight, on a dry basis, of the beverage. Also, the food or beverage of the present invention may be taken so that the effective ingredient contained therein is taken in an amount of, for example, preferably from 0.001 mg to 10 g/kg weight, and more preferably from 0.1 mg to 1 g/kg weight, per day for adult.

In addition, the present invention provides a feed for an organism, in which the extract of the present invention is contained, added and/or diluted. In still another embodiment, the present invention also provides a method of feeding an organism, characterized by administering the extract of the present invention to the organism. In still yet another embodiment, the present invention provides an organism-feeding agent characterized in that the organism-feeding agent comprises the extract of the present invention.

In these inventions, the organisms are, for example, cultured or bred animals, pet animals, and the like. The cultured or bred animal is exemplified by livestock, laboratory animals, poultry, pisces, crustacea or shellfish. The feed is exemplified by a feed for sustenance of and/or amelioration in physical conditions. The organism-feeding agent is exemplified by immersion agents, feed additives, and beverage additives.

According to these inventions, the same effects can be expected to be exhibited as those of the above-mentioned therapeutic agent or prophylactic agent of the present invention, on the basis of the insulin-mimetic action or the inhibitory action for aldose reductase of the extract of the present invention used in the present invention, in the organism exemplified above to which these are applied. In other words, the feed or the like of the present invention can exhibit an effect for treating or preventing a disease accompanying an abnormality in an amount of insulin or insulin response or diabetic complications in the organism.

The extract of the present invention used in the present invention is usually administered in an amount of preferably from 0.01 to 2000 mg per day per 1 kg of the weight of a subject organism. The administration can be carried

out, for example, by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the extract of the present invention in the feed is not particularly limited. The content can be appropriately set in accordance with the purposes. For example, the content of the above-mentioned effective ingredient is preferably in a ratio of from 0.001 to 15% by weight, on a dry basis. The content of the effective ingredient of the present invention in the organism-feeding agent may be adjusted to the same level as the feed.

The process for preparing the feed of the present invention is not particularly limited, and its composition may be set in accordance with a general feed, as long as the extract of the present invention is contained in the feed prepared. The organism-feeding agent can also be prepared in the same manner.

The organism to which the present invention can be applied is not limited. The cultured or bred animals include livestock such as *Equus, Bos, Porcus, Ovis, Capra, Camelus,* and *Lama;* laboratory animals such as mice, rats, guinea pigs, and rabbits; poultry such as *Chrysolophus,* ducks, *Meleagris,* and *Struthioniformes;* and the pet animals include dogs, cats, and the like, so that the feed can be widely applied.

In the present invention, for example, by allowing a subject organism to take the feed comprising the extract of the present invention, or immersing a subject organism into a solution containing the extract of the present invention (for example, prepared by dissolving the above immersing agent in water), the physical conditions of the livestock, laboratory animals, poultry, pet animals or the like can be well sustained or ameliorated. These embodiments are one embodiment of the feeding method of an organism in the present invention.

In addition, the present invention can provide an agent for enhancing glucose uptake into a cell comprising the above-mentioned effective ingredient. The agent for enhancing glucose uptake may be the above-mentioned effective ingredient itself, or a composition comprising the above-mentioned effective ingredient. The agent for enhancing glucose uptake may be prepared by, for example, combining the above-mentioned effective ingredient with another component, for example, an insulin preparation, an agent for enhancing insulin secretion, an agent for improving insulin resistance, an agent for ameliorating postprandial hyperglycemia, an agent for an insulin-mimetic action or the like that is commonly known, which can be used for the same application as that of the effective ingredient, and forming into a form of reagent usually used according to the above-mentioned process for preparing the therapeutic agent or prophylactic agent. The content of the above-mentioned effective ingredient in the agent for enhancing glucose uptake is not particularly limited, as long as the content is in an amount so that the desired effects of the present invention can be exhibited in consideration of administration method, purpose of use or the like of the agent for enhancing glucose uptake. A typical content of the effective ingredient in the agent for enhancing glucose uptake is from 0.1 to 100% by weight or so, on a dry basis. Also, the amount of the agent for enhancing glucose uptake used is not particularly limited, as long as the desired effects of the present invention can be exhibited. Especially in the case where the agent for enhancing glucose uptake is used by administering to a living body, the agent for enhancing glucose uptake may be used preferably in an amount so that the effective ingredient can be administered within the dose range of the effective ingredient in the above-mentioned therapeutic agent or prophylactic agent. The agent for enhancing glucose uptake is useful in treating or preventing a disease requiring an enhancing action on glucose uptake into a cell for the treatment or prevention. The disease is exemplified by, for example, the above-mentioned disease requiring an insulin-mimetic action, as well as cardiac diseases, especially cardiac infarction and post-ischemic injury of the heart, and the like. In addition, since the agent for enhancing glucose uptake enhances glucose uptake by a cell, when the action is exhibited in a muscle cell, an enhancing action on muscles or an action on recovery from fatigue can be induced. Also, the agent for enhancing glucose uptake can be used for the manufacture of a food, beverage or feed for treating or preventing these diseases. The food, beverage, or feed can be used according to the above-mentioned food, beverage or feed of the present invention. In addition, the agent for enhancing glucose uptake is also useful in screening of drugs for the above-mentioned diseases requiring an enhancing action on glucose uptake into a cell for the treatment or prevention. Furthermore, the agent for enhancing glucose uptake is useful in studies on mechanisms of action on glucose uptake by the cell, or functional studies on physical changes in the cell and the like.

In addition, the present invention can provide an agent for inducing differentiation into an adipocyte comprising the above-mentioned effective ingredient. The precursor cell that can be induced to differentiate into an adipocyte by the agent for inducing differentiation is not particularly limited, as long as the cell is capable of differentiating into adipocytes. The precursor cell includes, for example, preadipocyte, fibroblast, mesenchymal stem cell and the like. The agent for inducing differentiation may be the above-mentioned effective ingredient itself, or a composition comprising the above-mentioned effective ingredient. The agent for inducing differentiation may be prepared by, for example, combining the above-mentioned effective ingredient with other component, for example, an insulin preparation, an agent for enhancing insulin secretion, an agent for improving insulin resistance, an agent for ameliorating postprandial hyperglycemia, an agent for an insulin-mimetic action or the like which is commonly known, which can be used for the same application as that of the effective ingredient, and forming into a form of reagent usually used according to the above-mentioned process for preparing the therapeutic agent or prophylactic agent. The content of the above-mentioned

effective ingredient in the agent for inducing differentiation is not particularly limited, as long as the content is in an amount so that the desired effects of the present invention can be exhibited in consideration of administration method, purpose of use or the like of the agent for inducing differentiation. A typical content of the effective ingredient in the agent for inducing differentiation into an adipocyte is usually from 0.1 to 100% by weight or so, on a dry basis. Also, the amount of the agent for inducing differentiation used is not particularly limited, as long as the desired effects of the present invention can be exhibited. Especially in the case where the agent for inducing differentiation is administered to a living body, the agent for inducing differentiation may be used preferably in an amount so that the effective ingredient can be administered within the dose range of the effective ingredient in the above-mentioned therapeutic agent or prophylactic agent. The agent for inducing differentiation is useful in treating or preventing a disease requiring an action of inducing differentiation into an adipocyte for the treatment or prevention. The disease is exemplified by, for example, the above-mentioned disease requiring an insulin-mimetic action, as well as gout, fatty liver, cholecystolithiasis, menoxenia, infertility, and the like. The agent for inducing differentiation can be used for the manufacture of a food, beverage or feed for treating or preventing these diseases. The food, beverage, or feed can be used according to the above-mentioned food, beverage or feed of the present invention. In addition, the agent for inducing differentiation is also useful in screening of drugs for diseases requiring an action of inducing differentiation into an adipocyte for the treatment or prevention. Furthermore, the agent for inducing differentiation is useful in studies on mechanisms of action of inducing differentiation into an adipocyte, or functional studies on physical changes in the cell and the like.

In addition, the present invention can provide an inhibitory agent for aldose reductase comprising the above-mentioned effective ingredient. The inhibitory agent may be the above-mentioned effective ingredient itself, or a composition comprising the above-mentioned effective ingredient. The inhibitory agent may be prepared by, for example, combining the above-mentioned effective ingredient with another component, for example, epalrestat or the like, which can be used for the same application as that of the effective ingredient, and forming into a form of reagent usually used according to the above-mentioned process for preparing the therapeutic agent or prophylactic agent. The content of the above-mentioned effective ingredient in the inhibitory agent is not particularly limited, as long as the content is in an amount so that the desired effects of the present invention can be exhibited in consideration of administration method, purpose of use or the like of the inhibitory agent. A typical content of the effective ingredient in the inhibitory agent for aldose reductase is from 0.1 to 100% by weight or so, on a dry basis. Also, the amount of the inhibitory agent used is not particularly limited, as long as the desired effects of the present invention can be exhibited. Especially in the case where the inhibitory agent is used by administering to a living body, the inhibitory agent may be used preferably in an amount so that the effective ingredient can be administered within the dose range of the effective ingredient in the above-mentioned therapeutic agent or prophylactic agent. The inhibitory agent is useful in treating or preventing a disease requiring an inhibitory action for aldose reductase for the treatment or prevention. The disease is exemplified by, for example, the above-mentioned diabetic complications. Also, the inhibitory agent can be used for the manufacture of a food, beverage or feed for treating or preventing these diseases. The food, beverage, or feed can be used according to the above-mentioned food, beverage or feed of the present invention. In addition, the inhibitory agent is also useful in screening of drugs for the above-mentioned diabetic complications. Furthermore, the inhibitory agent is useful in studies on mechanisms on polyol metabolism and diabetic complications, or functional studies on physical changes and the like.

In addition, the extract of the present invention can be used as a material for cosmetics. The extract can be used by mixing the extract with any substance that can be used as cosmetics. In general, the extract is mixed with water, an alcohol, a fat or oil, a fatty acid, glycerol, an inorganic salt, an antiseptic, a surfactant, a vitamin, an amino acid, a saccharide, or the like, so that the extract can be used in the form of lotion, milky lotion, cream or the like. The content of the extract of the present invention is preferably from 0.1 to 80% by weight or so on a dry basis of the cosmetics.

In addition, as another embodiment of the present invention, there is provided an inhibitory agent for aldose reductase, characterized in that the inhibitory agent comprises as an effective ingredient at least one compound selected from the group consisting of xanthoangelol H, isobavachalcone, bavachromanol, munduleaflavanone A, isobavachin, and prostratol F. As the process for preparing these effective ingredients, the effective ingredient can be obtained by a known process. In addition, as the process for preparing an inhibitory agent for aldose reductase, the inhibitory agent can be prepared in accordance with the manufacture of the above-mentioned therapeutic agent or prophylactic agent of the present invention.

No toxicity is found even when the above-mentioned effective ingredient used in the present invention is administered in an effective dose for the exhibition of its action in a living body. For example, in the case of oral administration, no cases of deaths are found even when a dry product of a 60(v/v)% aqueous ethanol solution extract *of Angelica keiskei* koidz. is administered to a mouse at 1g/kg weight in a single dose. In addition, no cases of deaths are found even when the above-mentioned effective ingredient is orally administered to a rat at 1 g/kg weight in a single dose.

<u>EXAMPLES</u>

The present invention will be described more concretely hereinbelow by means of the examples, but the present

invention is by no means limited to these descriptions. Unless specified otherwise, % in Examples all means % by volume (v/v).

Example 1

Forty milliliters of an extraction solvent (40%, 50%, 60%, 70%, or 80% aqueous ethanol solution, or 100% ethanol) was added to 2 g of a product prepared by powdering a lyophilized product of leaf and stem portions of *Angelica keiskei* koidz., and extraction was carried out at 25°C for 30 minutes. The supernatant after centrifugation was quantified for chalcones using HPLC. As the column, TSK gel ODS-80Ts QA (4.6 mm × 25 cm: manufactured by Tosoh Corporation) was used. As to the elution ratio of a solvent A (prepared by mixing distilled water and acetonitrile at a volume ratio of 4 to 1, containing 0.1% trifluoroacetic acid) and a solvent B (prepared by mixing distilled water and acetonitrile at a volume ratio of 1 to 9, containing 0.1 % trifluoroacetic acid), the ratio of the solvent B changed linearly from 0 to 100% in a period of from 0 to 45 minutes, and the ratio of the solvent B is kept at 100% for the subsequent 10 minutes. The elution rate was 1 mL/minute, the detection was carried out at 215 nm, and the column temperature was 30°C.

The results are shown in Table 1. As shown in Table 1, in the extractions of TB1 and TB2 from the leaf and stem portions of *Angelica keiskei* koidz., it was clarified that the extractions with a water-containing ethanol had a higher efficiency than the extraction with 100% ethanol. In addition, it was clarified that in the extractions from the leaf and stem portions of *Angelica keiskei* koidz. with xanthoangelol and 4-hydroxyderricin, the extraction with the 60%, 70%, and 80% aqueous ethanol solutions had higher efficiency than the extraction with 100% ethanol.

Table 1 Effects of Water-Containing Alcohol in Extraction of Chalcones from Leaf and Stem Portions from *Angelica keiskei* koidz.

| | Amount of Chalcone Extracted (%) | | | |
|---|---|---|---|---|
| Extraction Solvent | Xanthoangelol | 4-Hydroxyderricin | TB1 | TB2 |
| 40% Aqueous Ethanol Solution | 19 | 30 | 225 | 163 |
| 50% Aqueous Ethanol Solution | 69 | 78 | 239 | 167 |
| 60% Aqueous Ethanol Solution | 101 | 106 | 220 | 164 |
| 70% Aqueous Ethanol Solution | 107 | 112 | 206 | 146 |
| 80% Aqueous Ethanol Solution | 109 | 111 | 184 | 144 |
| 100% Ethanol | 100 | 100 | 100 | 100 |

The amount extracted with 100% ethanol was expressed as 100%.

Example 2

A product prepared by powdering a lyophilized product of a root portion of *Angelica keiskei* koidz. was subjected to extraction and the chalcones in the extract were quantified, in the same manner as in Example 1. The results are shown in Table 2. In the extractions of TB1 and TB2 from the root portion of *Angelica keiskei* koidz., it was clarified that the extractions with a water-containing ethanol had a higher efficiency than the extraction with 100% ethanol. In addition, it was clarified that in the extractions from the root portion of *Angelica keiskei* koidz. with xanthoangelol and 4-hydroxyderricin, the 60%, 70%, and 80% aqueous ethanol solutions had higher efficiency than the extraction with 100% ethanol.

Table 2 Effects of Water-Containing Alcohol in Extraction of Chalcones from Root Portion from *Angelica keiskei* koidz.

| | Amount of Chalcone Extracted (%) | | | |
|---|---|---|---|---|
| Extraction Solvent | Xanthoangelol | 4-Hydroxyderricin | TB1 | TB2 |
| 40% Aqueous Ethanol Solution | 38 | 49 | 238 | 143 |
| 50% Aqueous Ethanol Solution | 92 | 94 | 251 | 144 |
| 60% Aqueous Ethanol Solution | 114 | 113 | 247 | 138 |
| 70% Aqueous Ethanol Solution | 111 | 111 | 249 | 142 |
| 80% Aqueous Ethanol Solution | 112 | 112 | 229 | 134 |
| 100% Ethanol | 100 | 100 | 100 | 100 |

The amount extracted with 100% ethanol was expressed as 100%.

Example 3 Preparation of Extract from Leaf and Stem Portions of *Angelica keiskei* koidz.

Forty milliliters of an extraction solvent (40%, 50%, 60%, 70%, or 80% aqueous ethanol solution, or 100% ethanol) was added to 2 g of a product prepared by powdering a lyophilized product of leaf and stem portions of *Angelica keiskei* koidz., and extraction was carried out at room temperature for 30 minutes. Thirty milliliters of the supernatant after centrifugation was concentrated to dryness, and the residue was dissolved in 0.75 mL of dimethyl sulfoxide.

Example 4 Preparation of Extract from Root Portion *of Angelica keiskei* koidz.

Forty milliliters of an extraction solvent (40%, 50%, 60%, 70%, or 80% aqueous ethanol solution, or 100% ethanol) was added to 2 g of a product prepared by powdering a lyophilized product of a root portion *of Angelica keiskei* koidz., and extraction was carried out at room temperature for 30 minutes. Thirty milliliters of the supernatant after centrifugation was concentrated to dryness, and the residue was dissolved in 0.75 mL of dimethyl sulfoxide.

Example 5 Activity for Inducing Differentiation into Adipocytes by Extract from Leaf and Stem Portions of *Angelica keiskei* koidz.

(1) Induction of Differentiation into Adipocytes

The induction of differentiation into adipocytes was carried out by partially modifying the method of Rubin C. S. et al. *(J. Biol. Chem.,* Vol. 253, No. 20, p7570-7578 (1978)). Preadipocyte cell line 3T3-L1 (ATCC CCL-92.1) was suspended in a 10% fetal bovine serum (manufactured by GIBCO)-containing Dulbecco's modified Eagle's medium (manufactured by Sigma, D6046) containing 200 μM ascorbic acid (hereinafter referred to as A-D-MEM medium) so as to have a density of $4 \times 10^3$ cells/mL, and the suspension was put in each well of a 12-well microtiter plate in an amount of 2 mL per well. The cells were cultured at 37°C for 7 days in the presence of 5% carbon dioxide gas. The medium was exchanged with the same medium on the second day and the fourth day. On the seventh day, the medium was exchanged with A-D-MEM medium containing 0.25 μM dexamethasone. Thereafter, to each well was put the dimethyl sulfoxide solution of the extract from the leaf and stem portions of *Angelica keiskei* koidz. with the 40% aqueous ethanol solution so as to have a final concentration of 0.1% or 0.033%, the dimethyl sulfoxide solution of the extract with the 50% aqueous ethanol solution so as to have a final concentration of 0.1%, 0.033% or 0.011%, or the dimethyl sulfoxide solution of the extract with the 60%, 70%, or 80% aqueous ethanol solution or the extract with the 100% ethanol so as to have a final concentration of 0.2%, 0.067% or 0.022%, each extract being prepared in Example 3. Here, there were set a group with addition of 4 μL of a 5 mg/mL aqueous solution of insulin (manufactured by TAKARA BIO INC.) as a positive control, and a group with addition of dimethyl sulfoxide as a negative control. After 45 hours, the medium was exchanged with A-D-MEM medium. The dimethyl sulfoxide solution of the extract from the leaf and stem portions *of Angelica keiskei* koidz. with the 40% aqueous ethanol solution so as to have a final concentration of 0.1% or 0.033 %, the dimethyl sulfoxide solution of the extract with the 50% aqueous ethanol solution so as to have a final concentration of 0.1%, 0.033% or 0.011%, the dimethyl sulfoxide solution of the extract with the 60%, 70%, or 80% aqueous ethanol solution or the extract with the 100% ethanol so as to have a final concentration of 0.2%, 0.067% or 0.022%, 2 μL of a 5 mg/mL aqueous solution of insulin as a positive control, or dimethyl sulfoxide as a negative control was put to each well. The cells were cultured for additional 7 days. The medium was exchanged on the second day and the fourth day, and at that time the dimethyl sulfoxide solution of the extract from the leaf and stem portions *of Angelica keiskei* koidz. with the 40% aqueous ethanol solution so as to have a final concentration of 0.1% or 0.033%, the dimethyl sulfoxide solution of the extract with the 50% aqueous ethanol solution so as to have a final concentration of 0.1%, 0.033% or 0.011%, the dimethyl sulfoxide solution of the extract with the 60%, 70%, or 80% aqueous ethanol solution or the extract with the 100% ethanol so as to have a final concentration of 0.2%, 0.067% or 0.022%, 2 μL of a 5 mg/mL aqueous solution of insulin as a positive control, or dimethyl sulfoxide as a negative control was put to each well.

(2) Determination of Amount of Biosynthesis of Triglyceride

The amount of triglyceride in the adipocytes was determined as an index for inducing differentiation into matured adipocytes, and as evaluation of insulin-mimetic action. After the termination of the culture, the medium was removed, and the cells were washed twice with a phosphate buffered saline. One milliliter of a solvent of hexane : isopropanol = 3 : 2 was added thereto. The mixture was allowed to stand at room temperature for 30 minutes, and thereafter the supernatant was collected. The procedures were repeated again, and 2 mL of the resulting supernatant was concentrated to dryness. The precipitate was dissolved in 100 μL of isopropanol, and thereafter the amount of triglyceride contained in 10 μL of the resulting solution was determined with Triglyceride E-Test (manufactured by Wako Pure Chemical Industries, Ltd., code 432-40201). All of the determinations were carried out twice.

As a result, induction of triglyceride biosynthesis could be also confirmed in the group with addition of the extract from the leaf and stem portions of *Angelica keiskei* koidz. with the 40%, 50%, 60%, 70%, or 80% aqueous ethanol solution or with the 100% ethanol, as compared to the group with addition of dimethyl sulfoxide, as well as in the group with addition of insulin. In other words, the activity of inducing differentiation into adipocytes was found in the extract from the leaf and stem portions of *Angelica keiskei* koidz. with the 40%, 50%, 60%, 70%, or 80% aqueous ethanol solution or with the 100% ethanol.

Example 6 Enhancing Action by Extract from Root Portion of *Angelica keiskei* koidz. on Glucose Uptake

(1) Preparation of Mature Adipocytes

The induction of differentiation into mature adipocytes was carried out by partially modifying the method of Rubin C. S. et al. 3T3-L1 cells were suspended in a 10% fetal bovine serum-containing Dulbecco's modified Eagle's medium containing 200 μM ascorbic acid so as to have a density of $4 \times 10^3$ cells/mL, and the suspension was put in each well of a 12-well microtiter plate in an amount of 2 mL per well. The cells were cultured at 37°C for 7 days in the presence of 5% carbon dioxide gas. On the seventh day, the medium was exchanged with 2 mL of a 10% fetal bovine serum-containing Dulbecco's modified Eagle's medium containing 200 μM ascorbic acid, 0.25 μM dexamethasone, 10 μg/mL insulin and 0.5 mM 3-isobutyl-1-methylxanthine (manufactured by Nacalai Tesque, Inc., 19624-44). After 45 hours, the medium was exchanged with 2 mL of a 10% fetal bovine serum-containing Dulbecco's modified Eagle's medium containing 200 μM ascorbic acid and 5 μg/mL insulin. The medium was exchanged on after 2 days and 4 days, and the cells were cultured for 7 days, to give mature adipocytes.

(2) Determination of Enhancing Action on Glucose Uptake into Mature Adipocytes

As the evaluation of enhancing action on glucose uptake, and also as the evaluation of insulin-mimetic action, the amount of 2-deoxyglucose uptake into mature adipocytes stimulated with the sample (the extract from root portion of *Angelica keiskei* koidz. prepared in Example 4) in the adipocytes was determined.

After the termination of the culture, the medium was removed, and the cells were washed twice with a 0.1% (w/v) bovine serum albumin (manufactured by Sigma, A8022)-containing Dulbecco's modified Eagle's medium. Thereafter, 1 mL of the same medium containing the dimethyl sulfoxide solution of the extract from the root portion of *Angelica keiskei* koidz. with the 40% or 50% aqueous ethanol solution at a final concentration of 0.1%, 0.067%, or 0.022%, the dimethyl sulfoxide solution of the extract from the root portion *of Angelica keiskei* koidz. with the 60% aqueous ethanol solution at a final concentration of 0.2%, 0.067%, or 0.022%, the dimethyl sulfoxide solution of the extract from the root portion of *Angelica keiskei* koidz. with the 70% or 80% aqueous ethanol solution or the dimethyl sulfoxide solution of the extract from the root portion of *Angelica keiskei* koidz. with the 100% ethanol so as to have a final concentration of 0.067% or 0.022%, each extract being prepared in Example 4, was put to each well. The cells were cultured overnight at 37°C in the presence of 5% carbon dioxide gas. Here, a group without addition of the sample was set as a negative control. After the overnight culture, the cells were washed twice with a HEPES buffered saline (140 mM NaCl, 5 mM KCl, 2.5 mM MgS04, 1 mM $CaCl_2$, 20 mM HEPES-Na (pH 7.4)), and 0.9 mL of the same buffer containing the dimethyl sulfoxide solution of the extract from the root portion *of Angelica keiskei* koidz. with the 40% or 50% aqueous ethanol solution at a final concentration of 0.1%, 0.067%, or 0.022%, the dimethyl sulfoxide solution of the extract from the root portion *of Angelica keiskei* koidz. with the 60% aqueous ethanol solution at a final concentration of 0.2%, 0.067%, or 0.022%, the dimethyl sulfoxide solution of the extract from the root portion *of Angelica keiskei* koidz. with the 70% or 80% aqueous ethanol solution or the dimethyl sulfoxide solution of the extract from the root portion *of Angelica keiskei* koidz. with the 100% ethanol so as to have a final concentration of 0.067% or 0.022%, was added thereto. The cells were cultured at 37°C for 75 minutes. At the point when 45 minutes passed, there was set in a well without addition of the sample a group with addition of insulin so as to have a final concentration of 1 μg/mL as a positive control. Thereafter, 100 μL of a HEPES buffered saline containing 0.5 μCi/mL 2-deoxy-[1,2-$^3$H(N)]-glucose (manufactured by PerkinElmer Life Sciences Inc., NET549A) and 1 mM 2-deoxyglucose (manufactured by Nacalai Tesque, Inc., 10722-11) was added thereto, and the cells were further cultured at 37°C for 10 minutes. After the termination of the culture, the supernatant was removed, the cells were washed three times with a phosphate buffer cooled to 4°C, and 0.5 mL of a 1% Nonidet P-40-containing phosphate buffer was added to lyse the cells, whereby 2-deoxy-[1,2-$^3$H(N)]-glucose taken into the cells was eluted. The radioactivity was determined with a liquid scintillation counter LS6500 (manufactured by Beckmann) using 25 μl of the supernatant with Ultima Gold (manufactured by PerkinElmer Life Sciences Inc., 6013329) as a scintillation cocktail.

As a result, enhancement of 2-deoxy-[1,2-$^3$H(N)]-glucose uptake was found in the group with addition of the extract from the root portion *of Angelica keiskei* koidz. with the 40%, 50%, 60%, 70%, or 80% aqueous ethanol solution or with the extract with the 100% ethanol as well as in the group with addition of insulin, as compared to the negative

control. In other words, enhancing activities for glucose uptake were found in the extract from the root portion *of Angelica keiskei* koidz. with the 40%, 50%, 60%, 70%, or 80% aqueous ethanol solution or the extract with the 100% ethanol.

Example 7 Induction of Differentiation into Adipocytes by Extract from Root Portion *of Angelica keiskei* koidz.

The inducing action on differentiation into mature adipocytes (insulin-mimetic action) of the extract from the root portion *of Angelica keiskei* koidz. with the 40%, 50%, 60%, 70%, or 80% aqueous ethanol solution or the extract with the 100% ethanol, prepared in Example 4 was determined in accordance with the method of Example 5.

Specifically, as a sample, there was set a group with addition of the dimethyl sulfoxide solution of the extract from the root portion *of Angelica keiskei* koidz. with the 40% aqueous ethanol solution at a final concentration of 0.1% or 0.033%, the dimethyl sulfoxide solution of the extract from the root portion *of Angelica keiskei* koidz. with the 50% aqueous ethanol solution at a final concentration of 0.033% or 0.011%, the dimethyl sulfoxide solution of the extract from the root portion *of Angelica keiskei* koidz. with the 70% aqueous ethanol solution at a final concentration of 0.022%, the dimethyl sulfoxide solution of the extract from the root portion of *Angelica keiskei* koidz. with the 60% or 80% aqueous ethanol solution or the dimethyl sulfoxide solution of the extract with the 100% ethanol so as to have a final concentration of 0.067% or 0.022%, respectively, to each well. Here, there were set a group with addition of 4 μL of a 5 mg/mL aqueous solution of insulin (manufactured by TAKARA BIO INC.) as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

As a result, induction of biosynthesis of triglyceride was found in the groups with addition of the extract from the root portion of *Angelica keiskei* koidz. with the 40%, 50%, 60%, 70%, or 80% aqueous ethanol solution or the extract with the 100% ethanol. In other words, inducing actions on differentiation into mature adipocytes were found in the extract from the root portion of *Angelica keiskei* koidz. with the 40%, 50%, 60%, 70%, or 80% aqueous ethanol solution or the extract with the 100% ethanol.

Preparation Example 1 Preparation of Extracts from Leaf and Stem Portions and Root Portion of *Angelica keiskei* koidz.

Forty milliliters of water was added to 2 g of a product prepared by powdering a lyophilized product of leaf and stem portions and a root portion of *Angelica keiskei* koidz., and extraction was carried out at room temperature for 30 minutes. Thirty milliliters of the supernatant after centrifugation was concentrated to dryness, and the residue was dissolved in 0.75 mL of water, to give extracts from leaf and stem portions and root portion of *Angelica keiskei* koidz.

Example 8 Inhibitory Action for Aldose Reductase of Extract from Root Portion of *Angelica keiskei* koidz.

Inhibitory action for aldose reductase of the extract from the root portion *of Angelica keiskei* koidz. was determined in accordance with the following method. As a sample, 20 μL of a 100 mM methylglyoxal solution was added to 10 μL of each of the kinds of ethanol extracts from the root portion of *Angelica keiskei* koidz. prepared in Example 4, and 10 μL of the water extract from the root portion of *Angelica keiskei* koidz. prepared in Preparation Example 1 (a solution prepared by dissolving the extract in a 50% aqueous dimethyl sulfoxide solution), 100 μL of 0.2 M phosphate buffer (pH 6.2), 20 μL of 1 mM NADPH (phosphate buffer), and 10 μL of a human muscle cell-derived aldose reductase solution (0.1 U/mL, manufactured by Wako Pure Chemical Industries, Ltd., phosphate buffer). For a period of 180 seconds after 30 seconds passed, the change in absorbance of NADPH at 340 nm was determined. As a negative control, a 50% aqueous dimethyl sulfoxide solution was used in place of the sample. Also, the absorbance was determined using distilled water as a blank solution for each sample in place of the methylglyoxal solution. The determination values were expressed as an average of two experimental values. The inhibitory ratio (%) for aldose reductase was calculated in accordance with the following formula:

$$\text{Inhibitory Ratio (\%)} = [1 - (\Delta As - \Delta Asb) / (\Delta Ac - \Delta Acb)] \times 100,$$

wherein ΔAs and ΔAc show a change in the absorbance of each of the sample solution and the negative control solution per minute; and ΔAsb and ΔAcb show a change in the absorbance of the blank solutions for the sample solution and the negative control solution per minute.

The sample was added in an amount so as to have a final concentration as shown in Table 3. As shown in Table 3, it was clarified that the extracts from the root portion *of Angelica keiskei* koidz. with the water-containing ethanols show higher inhibitory actions for aldose reductase than the water extract or the ethanol extract.

Table 3

| Extraction Solvent | Inhibitory Ratio (%) for Aldose Reductase | | |
|---|---|---|---|
| | 0.025% | 0.05% | 0.1% |
| Water | 32.8 | 46.7 | 63.6 |
| 40% Aqueous Ethanol Solution | 35.5 | 53.7 | 72.5 |
| 50% Aqueous Ethanol Solution | 40.8 | 57.0 | 72.6 |
| 60% Aqueous Ethanol Solution | 41.4 | 57.0 | 72.4 |
| 70% Aqueous Ethanol Solution | 39.6 | 57.5 | 73.9 |
| 80% Aqueous Ethanol Solution | 33.7 | 49.6 | 69.9 |
| 100% Ethanol | 16.6 | 28.3 | 47.6 |

Example 9 Inhibitory Action for Aldose Reductase of Extract from Leaf and Stem Portions of *Angelica keiskei* koidz.

The inhibitory activity for aldose reductase of the extract from the leaf and stem portions *of Angelica keiskei* koidz. prepared in Example 3 was determined in the same manner as in Example 8. The results are shown in Table 4. As shown in Table 4, it was clarified that the extract from the leaf and stem portions *of Angelica keiskei* koidz. with a 60% aqueous ethanol solution shows a higher inhibitory activity for aldose reductase than the water extract and the ethanol extract.

Table 4

| Extraction Solvent | Inhibitory Ratio (%) for Aldose Reductase | | |
|---|---|---|---|
| | 0.025% | 0.05% | 0.1% |
| Water | 22.5 | 39.5 | 55.6 |
| 60% Aqueous Ethanol Solution | 32.5 | 49.8 | 64.6 |
| 100% Ethanol | 1.4 | 5.2 | 17.6 |

Example 10 Preparation of Xanthoangelol H, Isobavachalcone, Bavachromanol, Munduleaflavanone A, Isobavachin, and Prostratol F

The extracts from the leaf and stem portions *of Angelica keiskei* koidz. with 40%, 50%, 60%, 70% and 80% ethanol solutions prepared in Example 3, and the extracts from the root portion *of Angelica keiskei* koidz. with 40%, 50%, 60%, 70% and 80% ethanol solutions prepared in Example 4 were subjected to analysis according to HPLC under the conditions shown in Example 1. As a result, it was confirmed that xanthoangelol H, isobavachalcone, bavachromanol, munduleaflavanone A, isobavachin, and prostratol F were contained in these extracts. These compounds were isolated by using various kinds of chromatographies.

Example 11 Inhibitory Activities for Aldose Reductase Contained in Extracts from Root Portion of *Angelica keiskei* koidz.

Inhibitory activities for aldose reductase of xanthoangelol H, isobavachalcone, bavachromanol, munduleaflavanone A, isobavachin, and prostratol F prepared in Example 10 were determined in the same manner as in Example 8.

The results are shown in Table 5. As shown in Table 5, it was clarified that xanthoangelol H, isobavachalcone, bavachromanol, munduleaflavanone A, isobavachin, and prostratol F have inhibitory activities for aldose reductase in a concentration-dependent manner.

In Examples 1 and 2, it was confirmed that TB1 and TB2 were contained in the water-containing ethanol extracts, and the present inventors have clarified that the inhibitory actions for aldose reductase are found in TB1 and TB2 in WO 2004/031165. In other words, it was clarified that there are many substances having inhibitory actions for aldose reductase other than TB1 and TB2 in the water-containing ethanol extracts shown in Examples 1 and 2.

Table 5

| Sample | Inhibitory Ratio (%) of Aldose Reductase | | | | |
|---|---|---|---|---|---|
| | 2.5 μM | 5 μM | 10 μM | 20 μM | 40 μM |
| Xanthoangelol H | 40.4 | 57.8 | 72.2 | 80.8 | N. T. |
| Isobavachalcone | N. T. | N. T. | 30.2 | 47.4 | 60.1 |
| Bavachromanol | 42.1 | 58.2 | 70.5 | 80.1 | N.T. |
| Munduleaflavanone A | 38.5 | 54.6 | 68.5 | 73.8 | N.T. |
| Isobavachin | 45.5 | 61.3 | 72.3 | 80.4 | N.T. |
| Prostratol F | 27.4 | 46.3 | 57.1 | 61.4 | N.T. |

N. T. is not tested.

Reference Example 1 Activities of Inducing Differentiation into Adipocytes by Xanthoangelol, 4-Hydroxyderricin, TB1, TB2, Xanthoangelol H,

Isobavachalcone, Munduleaflavanone A, Isobavachin, and Prostratol F

The inducing actions on differentiation into mature adipocytes (insulin-mimetic action) of xanthoangelol, 4-hydroxyderricin, TB1, TB2, xanthoangelol H, isobavachalcone, munduleaflavanone A, isobavachin, and prostratol F was determined in accordance with the method of Example 5.

Specifically, as a sample, there was set a group with addition of the dimethyl sulfoxide solution of xanthoangelol (final concentration: 1,3,10 μM), 4-hydroxyderricin (final concentration: 3, 10 μM), TB1 (final concentration: 3, 10 μM), TB2 (final concentration: 3, 10 μM), isobavachalcone (final concentration: 3,10 μM), xanthoangelol H (final concentration: 1.3, 4, 13 μM), munduleaflavanone A (final concentration: 3, 10 μM), isobavachin (final concentration: 3, 10, 30 μM), or prostratol F (final concentration: 3, 10 μM), respectively, to each well. Here, there were set a group with addition of 4 μL of a 5 mg/mL aqueous solution of insulin (manufactured by TAKARA BIO INC.) as a positive control, and a group with addition of dimethyl sulfoxide as a negative control. Thereafter, the medium and the sample were exchanged in the same manner as in the method described in Example 5, and after seven days from the addition of the sample, the amount of triglyceride in the adipocytes was determined.

As a result, induction of biosynthesis of triglyceride was found in the groups with addition of xanthoangelol, 4-hydroxyderricin, TB1, TB2, xanthoangelol H, isobavachalcone, munduleaflavanone A, isobavachin, and prostratol F. In other words, inducing actions on differentiation into mature adipocytes were found in xanthoangelol, 4-hydroxyderricin, TB1, TB2, xanthoangelol H, isobavachalcone, munduleaflavanone A, isobavachin, and prostratol F.

Reference Example 2 Enhancing Action by Xanthoangelol, 4-Hydroxyderricin, Xanthoangelol H and Isobavachalcone on Glucose Uptake

The amount of 2-deoxyglucose uptake into mature adipocytes stimulated with the sample in the adipocytes was determined in accordance with the method described in Example 6 as the evaluation of enhancing action of xanthoangelol, 4-hydroxyderricin, xanthoangelol H and isobavachalcone on glucose uptake and also as the evaluation of insulin-mimetic action.

Specifically, as a sample, there was set a group with addition of the dimethyl sulfoxide solution of xanthoangelol (final concentration: 3, 10 μM), 4-hydroxyderricin (final concentration: 3, 10, 30 μM), xanthoangelol H (final concentration: 30 μM), or isobavachalcone (final concentration: 3, 10, 30 μM) to each well. There were set a group without addition of the sample as a negative control, and a group with addition of insulin so as to have a final concentration of 1 μg/mL as a positive control. Thereafter, 2-deoxy-[1,2-$^3$H(N)]-glucose taken into the adipocytes was determined in the same manner.

As a result, enhancement of 2-deoxy-[1,2-$^3$H(N)]-glucose uptake was found in the groups with addition of xanthoangelol, 4-hydroxyderricin, xanthoangelol H and isobavachalcone as well as in the group with addition of insulin, as compared to the negative control. In other words, the activities of enhancing glucose uptake were found in xanthoangelol, 4-hydroxyderricin, xanthoangelol H and isobavachalcone.

Example 12

Forty kilograms of a dry powder *of Angelica keiskei* koidz. was added to 400 L of a 55% ethanol solution, and extraction was carried out at 25°C for 1 hour. Thereafter, the powder *of Angelica keiskei* koidz. was separated by filtration, and the resulting extract was concentrated under a reduced pressure with a concentration tank, to give 150 L of an

extract concentration from *Angelica keiskei* koidz. An excipient dextrin was added in an amount of 10 kg to the concentrate, and the mixture was lyophilized and powdered, to give 22 kg of an extract powder from *Angelica keiskei* koidz.

Example 13

The amount 2.5 kg of the extract powder from *Angelica keiskei* koidz. obtained in Example 12, 10.0 kg of crystalline cellulose, and 0.6 kg of a sucrose fatty acid ester were sequentially supplied to a mixer, and the mixture was then stirred for 15 minutes. The resulting mixture was formed into a tablet with a rotary tableting machine, to give 13.1 kg of a tablet. In addition, as a control, a tablet of a dry powder of *Angelica keiskei* koidz. was produced in the same manner except that the dry powder from *Angelica keiskei* koidz. described in Example 12 was used. Twenty members of panelists were subjected to sensory examination for these tablets. As a result, the tablet containing the extract from *Angelica keiskei* koidz. had lowered acerbity and astringency, so that the tablet was evaluated to be excellent in palatability.

Example 14

The amount 5.0 kg of the extract powder from *Angelica keiskei* koidz. obtained in Example 12, 5.0 kg of crystalline cellulose, 5.0 kg of lactose, and 0.7 kg of a sucrose fatty acid ester were sequentially supplied to a mixer, and the mixture was then stirred for 15 minutes. The resulting mixture was formed into a tablet with a rotary tableting machine, to give 15.7 kg of a tablet. In addition, as a control, a tablet of a dry powder from *Angelica keiskei* koidz. was produced in the same manner except that the dry powder *of Angelica keiskei* koidz. described in Example 12 was used. Twenty members of panelists were subjected to sensory examination for these tablets. As a result, the tablet containing the extract from *Angelica keiskei* koidz. had lowered acerbity and astringency, so that the tablet was evaluated to be excellent in palatability.

Example 15

The amount 2.5 kg of the extract powder from *Angelica keiskei* koidz. obtained in Example 12, 10.0 kg of crystalline cellulose, and 0.6 kg of a sucrose fatty acid ester were sequentially supplied to a mixer, and the mixture was stirred for 15 minutes. Further a 60% aqueous ethanol solution was added thereto to knead the mixture together, and the resulting mixture was granulated with an extrusion granulator. Furthermore, the granules were dried with a shelf-type hot air dryer at 60°C for 6 hours, and subjected to sizing with vibration sieves to give 13.1 kg of granulated products having sizes of from 20 to 100 mesh. In addition, as a control, a granulated product of a dry powder of *Angelica keiskei* koidz. was produced in the same manner except that the dry powder of *Angelica keiskei* koidz. described in Example 12 was used. Twenty members of panelists were subjected to sensory examination for these granulated products. As a result, the granulated product containing the extract from *Angelica keiskei* koidz. had lowered acerbity and astringency, so that the granulated product was evaluated to be excellent in palatability.

Example 16

The amount 2.0 kg of the extract powder from *Angelica keiskei* koidz. obtained in Example 12, 3.0 kg of lactose, 5.0 kg of trehalose, 3.5 kg of crystalline cellulose, 0.8 kg of a sucrose fatty acid ester, 0.5 kg of vitamin C, and 0.2 kg of citric acid were sequentially supplied to a mixer, and the mixture was stirred for 15 minutes. Further a 60% aqueous ethanol solution was added thereto to knead the mixture together, and the resulting mixture was granulated with an extrusion granulator. Furthermore, the granules were dried with a shelf-type hot air dryer at 60°C for 6 hours, and subjected to sizing with vibration sieves to give 15 kg of granulated products having sizes of from 20 to 100 mesh. In addition, as a control, a granulated product of a dry powder of *Angelica keiskei* koidz. was produced in the same manner except that the dry powder of *Angelica keiskei* koidz. described in Example 12 was used. Twenty members of panelists were subjected to sensory examination for these granulated products. As a result, the granulated product containing the extract from *Angelica keiskei* koidz. had lowered acerbity and astringency, so that the granulated product was evaluated to be excellent in palatability.

Example 17

The amount 2.9 kg of the extract powder from *Angelica keiskei* koidz. obtained in Example 12, 10.78 kg of lactose, 0.9 kg of a sucrose fatty acid ester, 0.1 kg of vitamin C, 0.07 kg of talc, and 0.25 kg of a flavor were sequentially supplied to a mixer, and the mixture was stirred for 15 minutes, to give 15 kg of a mixed powder. Thereafter, this mixed powder was packed in No. 1 Capsule, to give a capsule formulated with the extract powder from *Angelica keiskei* koidz.

Example 18

The amount 10.0 kg of soybean oil was placed in a mixer as a base oil, and 2.0 kg of the extract powder from *Angelica keiskei* koidz. obtained in Example 12, 1.0 kg of vitamin E, 1.0 kg of a glycerol fatty acid ester, and 0.8 kg of beeswax were added thereto, and the mixture was emulsified. The resulting emulsion was packed into a soft capsule comprising gelatin and glycerol as coating agents.

Example 19

A refreshing beverage containing an extract from *Angelica keiskei* koidz. was produced. The composition is as shown in Table 6.

Table 6 Refreshing Beverage Containing Extract from *Angelica keiskei* koidz. (each of % expressing w/w%)

| | |
|---|---|
| Extract Powder from *Angelica keiskei* koidz. Obtained in Example 12 (%) | 1 |
| Sugar (%) | 5 |
| 1/5 Lemon Juice(%) | 0.2 |
| Pectin (%) | 0.3 |
| Vitamin C (%) | 0.02 |
| Citric Acid (%) | 0.04 |
| Flavor (Lemon)(%) | 0.2 |
| Water | Balance |

The materials of Table 6 were sequentially mixed, and the mixture was homogenized. Thereafter, the homogenized mixture was thermally sterilized at 95°C for 15 seconds with a plate heater, and a 50 mL glass bottle was packed with the sterilized mixture. Thereafter, the mixture was sterilized at 75°C for 15 minutes with a pasteurizer.

In addition, as a control, a refreshing beverage containing a dry powder from *Angelica keiskei* koidz. was produced in the same manner except that the dry powder from *Angelica keiskei* koidz. described in Example 12. Twenty members of panelists were subjected to sensory examination for these refreshing beverages. As a result, the refreshing beverage containing the extract from *Angelica keiskei* koidz. had lowered acerbity and astringency, so that the refreshing beverage was evaluated to be excellent in palatability.

Example 20

A refreshing beverage containing an extract from *Angelica keiskei* koidz. was produced. The composition is as shown in Table 7.

Table 7 Refreshing Beverage Containing Extract from *Angelica keiskei* koidz. (each of % expressing w/w%)

| | |
|---|---|
| Extract Powder from *Angelica keiskei* koidz. Obtained in Example 12 (%) | 1 |
| Erythritol(%) | 5 |
| 1/5 Lemon Juice (%) | 0.2 |
| Pectin (%) | 0.3 |
| Vitamin C (%) | 0.02 |
| Citric Acid (%) | 0.04 |
| Flavor (Lemon) (%) | 0.2 |
| Water | Balance |

The materials of Table 7 were sequentially mixed, and the mixture was homogenized. Thereafter, the homogenized mixture was thermally sterilized at 95°C for 15 seconds with a plate heater, and a 50 mL glass bottle was packed with the sterilized mixture. Thereafter, the mixture was sterilized at 75°C for 15 minutes with a pasteurizer.

In addition, as a control, a refreshing beverage containing a dry powder from *Angelica keiskei* koidz. was produced in the same manner except that the dry powder from *Angelica keiskei* koidz. described in Example 12. Twenty members of panelists were subjected to sensory examination for these refreshing beverages. As a result, the refreshing beverage containing the extract from *Angelica keiskei* koidz. had lowered acerbity and astringency, so that the refreshing beverage was evaluated to be excellent in palatability.

Example 21

A refreshing beverage containing an extract from *Angelica keiskei* koidz. was produced. The composition is as shown in Table 8.

Table 8 Refreshing Beverage Containing Extract from *Angelica keiskei* koidz. (each of % expressing w/w%)

| | |
|---|---|
| Extract Powder from *Angelica keiskei* koidz. Obtained in Example 12 (%) | 0.2 |
| High Fructose Corn Syrup (%) | 6 |
| 1/5 Japanese Apricot *(Ume)* Juice (%) | 0.2 |
| Vitamin C (%) | 0.02 |
| Citric Acid (%) | 0.04 |
| Flavor (Japanese Apricot *(Ume))(%)* | 0.2 |
| Water | Balance |

The materials of Table 8 were sequentially mixed, and the mixture was homogenized. Thereafter, the homogenized mixture was thermally sterilized at 98°C for 15 seconds with a plate heater, and a 200 mL can was packed with the sterilized mixture in an amount of 190 g. Thereafter, the mixture was sterilized at 85°C for 15 minutes with a pasteurizer.

In addition, as a control, a refreshing beverage containing a dry powder from *Angelica keiskei* koidz. was produced in the same manner except that the dry powder from *Angelica keiskei* koidz. described in Example 12. Twenty members of panelists were subjected to sensory examination for these refreshing beverages. As a result, the refreshing beverage containing the extract from *Angelica keiskei* koidz. had lowered acerbity and astringency, so that the refreshing beverage was evaluated to be excellent in palatability.

Example 22

A refreshing beverage using a concentrate of the extract from *Angelica keiskei* koidz. obtained in Example 12 was produced. The composition is as shown in Table 9.

Table 9 Refreshing Beverage Containing Concentrate of Extract from *Angelica keiskei* koidz. (each of % expressing w/w%)

| | |
|---|---|
| Concentrate of Extract from *Angelica keiskei* koidz. Obtained in Example 12 (%) | 7 |
| Erythritol (%) | 5 |
| 1/5 Lemon Juice (%) | 0.2 |
| Pectin (%) | 0.3 |
| Vitamin C (%) | 0.02 |
| Citric Acid (%) | 0.04 |
| Flavor (Lemon) ((%) | 0.2 |
| Water | Balance |

The materials of Table 9 were sequentially mixed, and the mixture was homogenized. Thereafter, the homogenized mixture was thermally sterilized at 95°C for 15 seconds with a plate heater, and a 50 mL glass bottle was packed with the sterilized mixture. Thereafter, the mixture was sterilized at 75°C for 15 minutes with a pasteurizer.

In addition, as a control, a refreshing beverage containing a dry powder from *Angelica keiskei* koidz. was produced in the same manner except that the dry powder from *Angelica keiskei* koidz. described in Example 12. Twenty members of panelists were subjected to sensory examination for these refreshing beverages. As a result, the refreshing beverage containing the extract from *Angelica keiskei* koidz. had lowered acerbity and astringency, so that the refreshing beverage was evaluated to be excellent in palatability.

Example 23

Each of the refreshing beverages produced in the above-mentioned Examples 19 to 22 was packed in a 200 mL pouch with a closing plug, or a 100 mL retort pouch in place of the glass bottle or can, and sterilized in the same manner, to give each of pouched beverages.

INDUSTRIAL APPLICABILITY

According to the present invention, there are provided a water-containing extract derived from a plant belonging to Umbelliferae, and a process for preparing the extract. The extract richly contains natural product-derived physiologically active substances (for example, the above-mentioned substances showing insulin-mimetic action, inhibitory action for aldose reductase, and the like), and the extract is useful as a material for a medicament, food, beverage or feed which is effective for a disease accompanying an abnormality in an amount of insulin or insulin response and/or diabetic complications. Also, by ingesting the food or beverage as daily foodstuff, the disease accompanying an abnormality in an amount of insulin or insulin response and/or diabetic complications can be prevented, ameliorated or the like. Therefore, the functional foodstuff containing the extract of the present invention are functional foodstuff useful in maintaining homeostasis of a living body by their insulin-mimetic action and/or inhibitory action for aldose reductase. In addition, according to the present invention, there is provided an agent for insulin-mimetic agent comprising the extract of the present invention, and the agent for insulin-mimetic action is useful for functional studies on insulin and screening of a medicament for a disease associated with insulin. Further, according to the present invention, there is provided an agent for enhancing glucose uptake into a cell, comprising the extract of the present invention, and the agent for enhancing glucose uptake is useful for the prevention or treatment of a disease requiring an action for enhancing glucose uptake into a cell for the treatment or prevention, the manufacture of a food, beverage or feed for the treatment or prevention of the disease, and screening of a drug for a disease requiring an action for enhancing glucose uptake. Furthermore, according to the present invention, there is provided an agent for inducing differentiation into an adipocyte, comprising the extract of the present invention, and the agent for inducing the differentiation is useful for the prevention or treatment of a disease requiring an action of inducing differentiation into an adipocyte for the treatment or prevention, the manufacture of a food, beverage or feed for the treatment or prevention of the disease, and screening of a drug for a disease requiring an action of inducing the differentiation. Moreover, according to the present invention, there is provided an inhibitory agent for aldose reductase, comprising the extract of the present invention, and the inhibitory agent is useful for the prevention or treatment of a disease requiring an action of the inhibitory action for aldose reductase for the treatment or prevention, the manufacture of a food, beverage or feed for the treatment or prevention of the disease, and screening of a drug for a disease requiring the inhibitory action.

## Claims

1. An extract obtainable by extracting a plant belonging to Umbelliferae, or a processed product thereof, with a water-containing alcohol.

2. The extract according to claim 1, wherein the plant belonging to Umbelliferae is *Angelica keiskei* koidz.

3. The extract according to claim 1 or 2, wherein the water-containing alcohol is an aqueous ethanol solution having a concentration of 40(v/v)% or more and less than 100(v/v)%.

4. A process for preparing an extract derived from a plant belonging to Umbelliferae or a processed product thereof, comprising the steps of extracting a plant belonging to Umbelliferae, or a processed product thereof, with a water-containing alcohol.

5. The process according to claim 4, wherein the plant belonging to Umbelliferae is *Angelica keiskei* koidz.

6. The process according to claim 4 or 5, wherein the water-containing alcohol is an aqueous ethanol solution having a concentration of 40(v/v)% or more and less than 100(v/v)%.

7. A therapeutic agent or prophylactic agent for a disease accompanying an abnormality in an amount of insulin or insulin response and/or diabetic complications, **characterized in that** the therapeutic agent or prophylactic agent comprises as an effective ingredient the extract as defined in any one of claims 1 to 3.

8. An agent for an insulin-mimetic action, **characterized in that** the agent comprises as an effective ingredient the extract as defined in any one of claims 1 to 3.

9. A food, beverage or feed, **characterized in that** the food, beverage or feed comprises the extract as defined in any one of claims 1 to 3.

**10.** The food, beverage or feed according to claim 9, wherein the food, beverage or feed is used for treating or preventing a disease accompanying an abnormality in an amount of insulin or insulin response and/or diabetic complications.

**11.** An agent for enhancing glucose uptake into a cell, **characterized in that** the agent comprises as an effective ingredient the extract as defined in any one of claims 1 to 3.

**12.** An agent for inducing differentiation into an adipocyte, **characterized in that** the agent comprises as an effective ingredient the extract as defined in any one of claims 1 to 3.

**13.** An inhibitory agent for aldose reductase, **characterized in that** the agent comprises as an effective ingredient the extract as defined in any one of claims 1 to 3.

**INTERNATIONAL SEARCH REPORT**

International application No.
PCT/JP2004/008913

A. CLASSIFICATION OF SUBJECT MATTER
Int.Cl[7] A61K35/78, A61P3/08, 3/10, 5/48, 43/00, A23L1/30, A23K1/16, C12N9/99

According to International Patent Classification (IPC) or to both national classification and IPC

B. FIELDS SEARCHED

Minimum documentation searched (classification system followed by classification symbols)
Int.Cl[7] A61K35/78, A23L1/30, A23K1/16

Documentation searched other than minimum documentation to the extent that such documents are included in the fields searched

Electronic data base consulted during the international search (name of data base and, where practicable, search terms used)
CA(STN), MEDLINE(STN), BIOSIS(STN), EMBASE(STN), JICST(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | JP 9-67254 A (Yoshihide HAGIWARA), 11 March, 1997 (11.03.97), Claims 1 to 3; Par. No. [0008] (Family: none) | 1-6<br>7-13 |
| Y | Alison M. et al., Insulin-releasing and insulin-like activity of the traditional anti-diabetic plant Coriandrum sativum (coriander), British J. Nutrition, 1999, Vol.81, No.3, pages 203 to 209 | 1-13 |
| Y | JP 2002-80362 A (Kao Corp.), 19 March, 2002 (19.03.02), Par. Nos. [0001], [0012] (Family: none) | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 August, 2004 (30.08.04) | 14 September, 2004 (14.09.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/008913

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-138045 A (Ichimaru Pharcos Co., Ltd.), 14 May, 2002 (14.05.02), (Family: none) | 1-13 |
| Y | JP 10-295325 A (Katsuharu NAGAMITSU), 10 November, 1998 (10.11.98), Claim 4 (Family: none) | 1-13 |
| Y | JP 9-121810 A (Yugen Kaisha Hida Aroe), 13 May, 1997 (13.05.97), (Family: none) | 1-13 |
| Y | Keizo SEKIYA, "Ninjin ni Fukumareru Zenku Shibo Saibo no Bunka Chosetsu Seibun", Shokuhin Kenkyu Seika Joho, 1997, Vol.9, No.1, pages 24 to 25 | 1-13 |
| Y | JP 7-322847 A (Fujimaru Shokuhin Kabushiki Kaisha), 12 December, 1995 (12.12.95), Table 1 (Family: none) | 1-13 |
| Y | C.Leigh Broadhurst et al., Insulin-like Biological Activity of Culinary and Medicinal Plant Aqueous Extracts in Vitro, J.Agric.Food.Chem, 2000, Vol.48, No.3, pages 849 to 852, table 1 | 1-13 |
| Y | Yoshihito OKADA et al., Search for Naturally Occurring Substances for Prevention against the Complications of Diabetes-Inhibitation Effect on Aldose Reductase and Platelet Aggretion, Natural Medicine, 1994, Vol.48, No.4, pages 324 to 329 | 1-13 |
| Y | Ritsu KOSHO et al., Streptozotocin Tonyobyo Rat ni Okeru Kasanka Shishitsu Seisei ni Oyobosu Carotenoid Tenka Shiryo Toyo no Eikyo, The Japanese Society of Nutrition and Food Science Sokai Koen Yoshishu, 1998, Vol.53nd, page 245 | 1-13 |

Form PCT/ISA/210 (continuation of second sheet) (January 2004)